Europäisches Patentamt

European Patent Office

Office européen des brevets

(11) Veröffentlichungsnummer: **0 279 925**
B1

(12) EUROPÄISCHE PATENTSCHRIFT

(45) Veröffentlichungstag der Patentschrift:
12.09.90

(51) Int. Cl.⁵: **B29C 65/82**, C09J 5/02,
A61M 5/14

(21) Anmeldenummer: 87117798.6

(22) Anmeldetag: 02.12.87

(54) Verbindungsstück, insbesondere für medizinische Überleitungsgeräte.

(30) Priorität: 29.01.87  DE 3702612

(43) Veröffentlichungstag der Anmeldung:
31.08.88 Patentblatt 88/35

(45) Bekanntmachung des Hinweises auf die Patenterteilung:
12.09.90 Patentblatt 90/37

(84) Benannte Vertragsstaaten:
AT BE CH DE ES FR GB GR IT LI LU NL SE

(56) Entgegenhaltungen:
DD-C- 30 386
FR-A- 2 527 453
US-A- 3 740 290
US-A- 3 956 631

(73) Patentinhaber: B. Braun Melsungen AG, Carl-Braun
Strasse, D-3508 Melsungen(DE)

(72) Erfinder: Ried, Frank, Emdener Strasse 34,
D-3502 Vellmar(DE)

(74) Vertreter: Selting, Günther, Dipl.-Ing. et al,
Patentanwälte von Kreisler, Selting, Werner
Deichmannhaus am Hauptbahnhof, D-5000 Köln 1(DE)

ACTORUM AG

## Beschreibung

Die Erfindung bezieht sich auf Verbindungsstücke aus glasklarem Kunststoff mit einem hülsenförmigen Schlauchansatz zum Ein- oder Aufstecken eines mit dem Schlauchansatz mittels eines Klebe-/Lösungsmittels zu verbindenden Schlauches aus glasklarem Kunststoff, insbesondere für medizinische Überleitungsgeräte.

Bisher wird glasklarer PVC-Schlauch mit dem glasklaren Schlauchansatz eines Verbindungsstückes unter Einwirkung eines Klebe-/Lösungsmittels (z.B. THF) verbunden. Beide Teile sind vor und nach der Klebe-/Lösungsmitteleinwirkung glatt und glasklar. Folglich kann man optisch nicht erkennen, ob die zusammengesteckten Teile mit Klebe-/Lösungsmittel benetzt bzw. gleichmäßig benetzt wurden und die Verbindung dicht und fest ist.

Undichtigkeiten durch das vollständige oder teilweise Fehlen von Klebe-/Lösungsmittel im Bereich des Schlauchansatzes können bisher nur mit extrem hohen Prüfdrücken (>4 bar) nachgewiesen werden, da die Haftung zwischen den glatten Flächen des Schlauchansatzes und des Schlauches sehr stark ist und bei niedrigen Prüfdrücken nicht nachgibt. Die hohen Prüfdrücke jedoch sind nachteilig, weil sie Einzelteile der Leitungssysteme beschädigen können, so daß das Prüfobjekt für die Anwendung unbrauchbar wird. Beispielsweise kann der Schlauch aufgebläht werden, die Zuspritzmöglichkeiten können zerstört werden oder es können intakte Klebestellen Schaden erleiden. Ferner ist mit hohen Prüfdrücken nicht einmal eine genaue Aussage über die Dichtigkeit der Verbindung erzielbar, weil nach einer bestimmten Lagerzeit die mangelhaften Verbindungsstellen, die ohne Klebe-/Lösungsmittel zunächst dicht sind, durch Relaxation des Schlauches bereits bei niedrigen Nutzdrücken undicht werden bzw. sich während der Lagerung lösen. Die bisher praktizierten Dichtigkeitsprüfungen mit hohen Drücken sind also nicht nur aufwendig und kostenintensiv, sondern unzuverlässig und für das Produkt schädlich. Die Automatisierung solcher Druck-Dichtigkeitsprüfungen ist nur mit großem technischem Aufwand möglich, der sich angesichts des unzureichenden Ergebnisses nicht lohnt.

Der Erfindung liegt die Aufgabe zugrunde, Verbindungsstücke der eingangs erwähnten Art so auszubilden, daß durch zerstörungsfreie optische Kontrolle die Beschaffenheit der Verbindung zwischen Schlauchansatz und Schlauch geprüft werden kann.

Diese Aufgabe wird erfindungsgemäß dadurch gelöst, daß der Schlauchansatz auf der dem Schlauch zugewandten Fläche mattiert ist.

An den mattierten Flächen des Schlauchansatzes erhält der sonst glasklar erscheinende Kunststoff ein milchiges Aussehen und ist dort undurchsichtig. Wird nun ein Schlauch ohne Klebe-/Lösungsmittel mit dem Schlauchansatz zusammengesteckt, so verändert sich die Mattierung nicht, und das Fehlen des Klebe-/Lösungsmittels ist eindeutig erkennbar. Wird dagegen der mit Klebe-/Lösungsmittel benetzte Schlauch mit dem Schlauchansatz zusammengesteckt, so tritt durch das Anlösen des Kunststoffmateriales des Schlauchansatzes eine Glättung der mattierten Fläche derart ein, daß der Schlauchansatz an den Stellen, an denen Klebe-/Lösungsmittel eine homogene Verklebung bewirkt hat, glasklar wird. An mangelhaften bzw. undichten Klebestellen bleibt die Mattierung der Schlauchansatzfläche erhalten und es ist die Schadhaftigkeit der Verbindung ohne weiteres visuell erkennbar. Aus diesen Gegebenheiten lassen sich Rückschlüsse auf die Dichtigkeit und die Festigkeit der Schlauch-Schlauchansatz-Verbindung ziehen. Mangelhafte bzw. undichte Klebestellen können durch eine zerstörungsfreie Prüfung erkannt werden, die als Inprozeßkontrolle visuell oder mit Hilfe von technischen Geräten erfolgen kann. Beispielsweise ist es vorteilhaft, die fertig montierte Leitung an einem mit Durchlicht arbeitenden Meßgerät vorbeilaufen zu lassen. Es kann eine 100%ige Kontrolle kostengünstig durchgeführt werden, wodurch ein hoher Qualitätsstandard erreicht wird. Die bei korrekter Verklebung erzielte Glasklarheit von Schlauchansatz und Schlauch läßt auch erkennen, ob der Schlauch richtig mit dem Schlauchansatz zusammengesteckt wurde.

Dies hat gemeinsam mit der garantiert homogenen Verklebung hohe Zug- und Druckfestigkeit der Verbindung zur Folge. Zusatzstoffe werden für die Verklebung nicht benötigt, so daß keine Verminderung der Klebequalität oder Gefährdung des Patienten durch Toxizität zu befürchten ist. Da die zusammengesetzte Leitung zur Dichtigkeitskontrolle keinen hohen Prüfdrücken ausgesetzt wird, behalten der Schlauch und andere Einzelteile der Leitung ihre ursprünglichen Eigenschaften und die Produktsicherheit ist sehr hoch. Wenn zusätzlich zu der durch die Umwandlung von matt in glasklar erreichten Indikation des Verklebungszustandes eine Dichtigkeitsprüfung vorgenommen werden soll, genügen zu diesem Zweck niedrige Prüfdrücke, weil eventuelle Undichtigkeiten bereits bei geringen Drücken auftreten, da zwischen der mattierten Fläche des Schlauchansatzes und der glatten Fläche des Schlauches nur geringe Haftkräfte wirken können.

In vorteilhafter Ausgestaltung der Erfindung ist vorgesehen, daß die Mattierung aus einer gleichmäßigen Oberflächenaufrauhung gebildet ist. Die Aufrauhung kann vorteilhafterweise dadurch erreicht werden, daß die die betreffenden Flächen des Schlauchansatzes formenden Flächen eines Spritzgießwerkzeuges nicht poliert bzw. z.B. mittels Sandstrahlen oder ähnlicher Verfahren leicht strukturiert worden sind. Es sollen nur diejenigen Flächen des Verbindungsstückes aufgerauht sein, die später bei der Verklebung mit dem Klebe-/Lösungsmittel in Berührung kommen sollen. Auf diese Weise wird gewährleistet, daß bei korrektem Sitz des Schlauches und homogener Klebeverbindung das Endprodukt insgesamt glasklar ist. Bei einem Verbindungsstück mit konzentrischen inneren und äußeren Schlauchansätzen, die das Schlauchende in einem Ringspalt zwischen sich aufnehmen und die beide mit ihm verklebt werden sollen, sind die Innenfläche des äußeren Schlauchansatzes und die Außenfläche des inneren Schlauchansatzes aufgerauht. Bei Einschieben des mit Klebe-/Lösungsmittel benetzten Schlauchendes in den Ringspalt zwischen den beiden Schlauchansätzen werden die Innenfläche des einen Schlauchansatzes und die Außenfläche des anderen Schlauchansatzes angelöst und der Mattierungseffekt verschwindet. Die beiden Schlauchansätze werden ganz oder teilweise glasklar und zeigen die Beschaffenheit des Verbundes der Teile optisch eindeutig an.

In der Zeichnung sind Ausführungsbeispiele der Erfindung schematisch dargestellt. Es zeigen:

Fig. 1 eine Draufsicht auf ein Verbindungsstück für zwei Schläuche unterschiedlicher Durchmesser vor der Verbindung,

Fig. 2 einen Querschnitt längs der Linie 2-2 in Figur 1,

Fig. 3 einen Längsschnitt durch die zusammengesteckte Anordnung gemäß Figur 1 vor der Verklebung,

Fig. 4 eine Draufsicht auf die Anordnung nach Figur 3 nach der Verklebung ohne Fehlerstellen,

Fig. 5 eine Draufsicht auf die Anordnung nach Figur 3 nach der Verklebung mit Fehlerstellen,

Fig. 6 eine teilweise aufgeschnittene Draufsicht auf ein Verbindungsstück für drei identische Schläuche und

Fig. 7 eine Draufsicht auf eine Tropfkammer eines Transfusionsgerätes mit angesetztem Überleitungsschlauch.

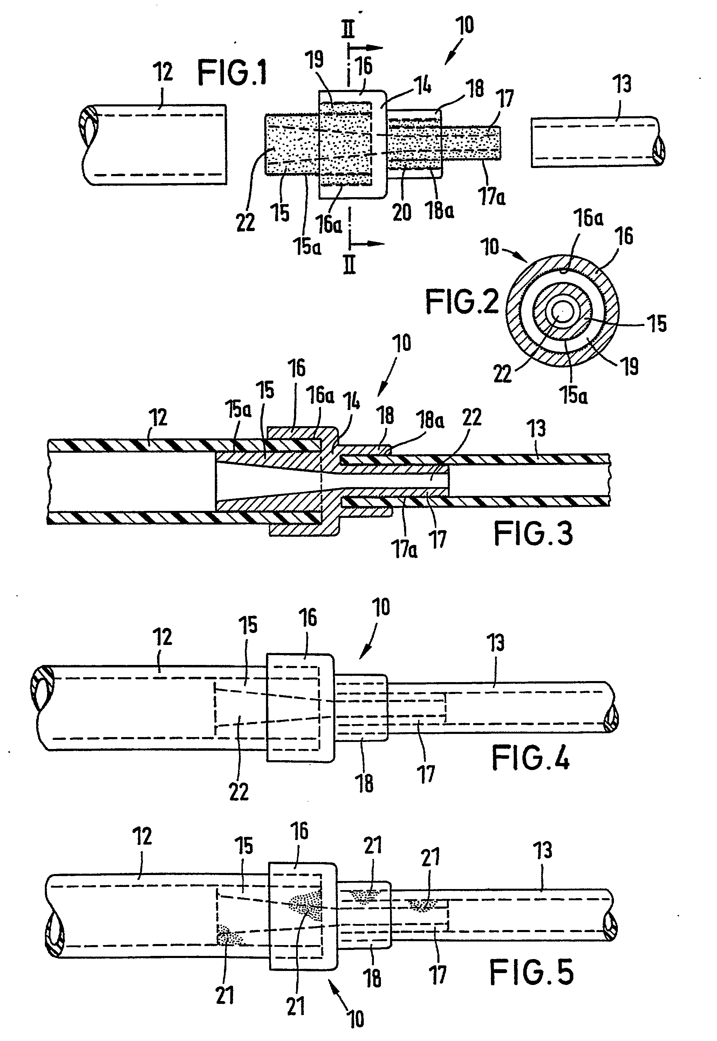

Ein Verbindungsstück 10 ist aus glasklarem, steifem Kunststoff hergestellt und dient zur koaxialen Verbindung eines Schlauches 12 mit größerem Durchmesser und eines Schlauches 13 mit kleinerem Durchmesser. Die beiden Schläuche 12 und 13 sind aus glasklarem PVC-Kunststoffmaterial gefertigt und haben eine gewisse Flexibilität. Das Verbindungsstück 10 besitzt eine zentrale Bodenplatte 14, von der nach beiden Seiten Schlauchansätze abgehen. Bei dem Beispiel nach Fig. 1 bis 5 ist vorgesehen, daß die Enden der beiden Schläuche 12 und 13 jeweils zwischen einem inneren Schlauchansatz 15 bzw. 17 und einem äußeren Schlauchansatz 16 bzw. 18 aufgenommen werden. Die entsprechenden Ringräume 19 bzw. 20 zwischen den Schlauchansätzen 15, 16 bzw. 17, 18 sind in radialer Richtung so bemessen, daß sie die Enden der Schläuche 12 und 13 im wesentlichen passend aufnehmen, so daß die einander zugewandten Flächen dieser Teile sich berühren. Sämtliche Schlauchansätze 15 bis 18 sind zueinander koaxial an der Bodenplatte 14 angebracht. Ein zentraler Kanal 22 erstreckt sich durch die beiden inneren Schlauchansätze 15 und 17. Er ist in dem inneren Schlauchansatz 17 zylindrisch und erweitert sich durch die Bodenplatte 14 hindurch konisch gegen das Ende des inneren Schlauchansatzes 15. Diese Gestaltung des Kanals 22 ist aus strömungstechnischen Gründen günstig.

Die zylindrischen Innenflächen 16a und 18a der äußeren Schlauchansätze 16 und 18 sowie die zylindrischen Außenflächen 15a und 17a der inneren Schlauchansätze 15 und 17 sind mattiert. Dies ist in der Zeichnung durch Rasterung bzw. Konturenzäckelung angedeutet. Die Mattierung der betreffenden Flächen 15a, 16a, 17a, 18a kann vorteilhafterweise durch Herstellung des Schlauchverbindungsstückes 10 mit einem Spritzgießwerkzeug erreicht werden, das z.B. durch Sandstrahlbehandlung an den infragekommenden Stellen gleichmäßig so strukturiert ist, daß der Spritzgußformling eine feine Oberflächenaufrauhung erhält. Wenn die Enden der beiden Schläuche 12 und 13 ohne Klebe-/ Lösungsmittel mit dem Verbindungsstück 10 zusammengesteckt werden, bleibt durch die glasklaren Schläuche 12,13 die gleichmäßige Aufrauhung der Oberflächen der Schlauchansätze 15 bis 18 als Mattierung sichtbar und es ist ohne weiteres optisch erkennbar, daß das Klebe-/ Lösungsmittel fehlt und die Verbindung nicht dicht sein kann. Dieser unerwünschte Zustand ist in Figur 3 veranschaulicht.

Wenn bei ordnungsgemäßer Klebemittel-Benetzung der Innen- und Außenflächen der Enden der beiden Schläuche 12 und 13 diese bis zum Anschlag gegen die beiden Flächen der Bodenplatte 14 zwischen die inneren und äußeren Schlauchansätze 15,17 und 16,18 geschoben sind, wird das Kunststoffmaterial der Schlauchansätze 15 bis 18 von dem Klebe-/Lösungsmittel angelöst und die aufgerauhten Flächen 15a bis 18a werden geglättet. Die Glättung hat zur Folge, daß das Verbindungsstück 10 überall dort glasklar transparent wird, wo sich Klebe-/Lösungsmittel befindet. Bei einer homogenen Benetzung aller zur gegenseitigen Anlage kommenden Flächen von Verbindungsstück 10 und Schläuchen 12 bzw. 13 ergibt sich ein schadstellenfreies glasklares Erscheinungsbild der Anordnung - wie in Figur 4 veranschaulicht ist.

Mangelhafte Klebestellen, die zu Undichtigkeiten und verringerter Zugfestigkeit der Verbindung führen, sind optisch dadurch erkennbar, daß mattierte Stellen 21 in der im übrigen glasklaren Anordnung zurückbleiben. Dies ist in Figur 5 dargestellt, die ein Ausschußteil zeigt. Eine Inprozeßkontrolle kann visuell oder mit Hilfe eines mit Durchlicht arbeitenden Meßgerätes vorgenommen werden. Da hohe Prüfdrücke zur Ermittlung der Dichtigkeit der Verbindung nicht zur Anwendung kommen, ist die Produktsicherheit groß. Wenn zusätzlich zu der optischen Kontrolle eine Dichtigkeitsprüfung unter Druckbedingungen durchgeführt wird, zeigen sich die Undichtigkeiten an den Stellen 21 schon bei niedrigen Drücken, da zwischen den aufgerauhten Stellen 21 der Schlauchansätze 15 bis 18 und den glatten Flächen der Schläuche 12 und 13 nur geringe Haftkräfte wirksam sind. Eine Beschädigung des Produktes durch Prüfdrücke ist deshalb ausgeschlossen, und es wird ein hoher Qualitätsstandard gewährleistet.

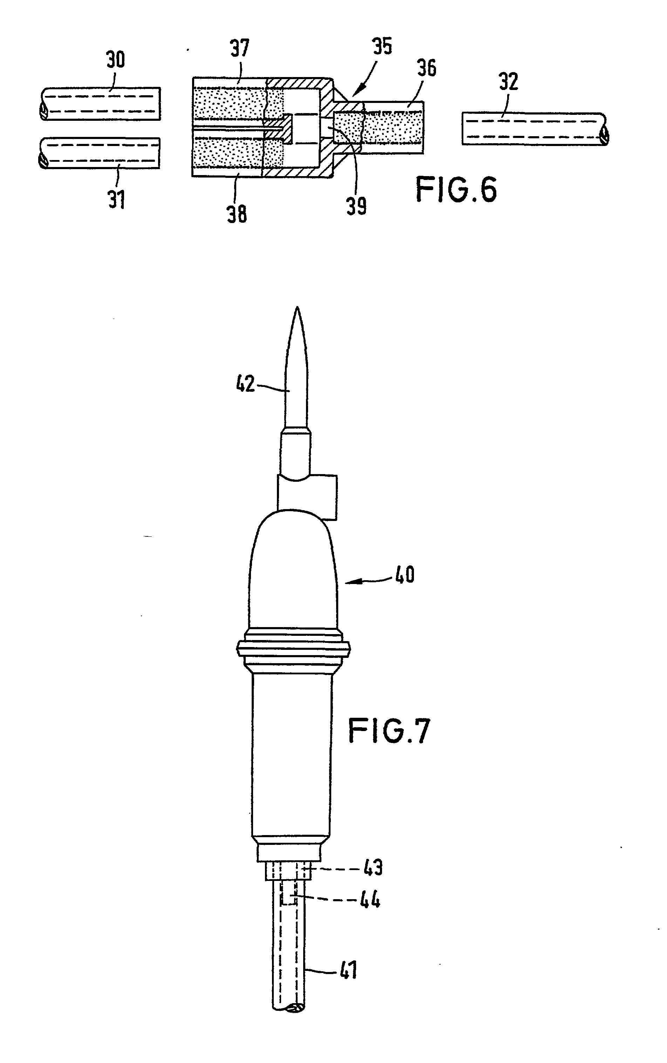

Das Prinzip der Flächenaufrauhung als Mittel zur Sichtbarmachung von Verklebungsfehlern kann bei allen glasklaren Produkten mit Schlauchansatz angewendet werden. Weitere Beispiele sind in den Figuren 6 und 7 gezeigt.

Bei dem Beispiel nach Figur 6 werden drei Schläuche 30, 31, 32 über ein Verbindungsstück 35 miteinander verbunden. Das Verbindungsstück 35 ist ein flacher Körper aus Kunststoff, der einen nach einer Seite gerichteten Schlauchansatz 36 und zwei nach der anderen Seite gerichtete zueinander parallele Schlauchansätze 37, 38 aufweist. Die drei Schlauchansätze 36,37,38 stehen über eine Öffnung 39 miteinander in Verbindung, so daß die Lumina der drei Schläuche 30,31,32 strömungsmäßig verbunden sind. Die Innenflächen der Schlauchansätze 36, 37, 38 sind - wie durch die Rasterung angedeutet ist - mattiert. Wenn die Schläuche 30,31,32 richtig eingeschoben sind und das Klebe-/Lösungsmittel (z.B. THF) homogen verteilt eine gleichmäßige Anlösung der mattierten Flächen und eine vollflächige Verklebung der Teile bewirkt, ist das Verbindungsstück 35 insgesamt glasklar und ein mit Durchlicht arbeitendes Meßgerät stellt fest, daß die fertig montierte Kupplung für den Anschluß mehrerer Infusions- oder Transfusionsleitungen 100%ig dicht ist. Die homogene Verklebung sorgt für hohe Druck- und Zugfestigkeit der Verbindung.

Bei dem Beispiel nach Figur 7 ist das untere Ende einer Tropfkammer 40 eines Transfusionsgerätes mit einem Überleitungsschlauch 41 fest verbunden. Die Tropfkammer 40 besteht einschließlich ihres Einstichdornes 42 aus glasklarem Kunststoff und an ihrem unteren Ende befinden sich ebenfalls glasklare Schlauchansätze 43,44, mit denen die Innen- und Außenfläche des Endes des Überleitungsschlauches 41 verklebt sind. Auch in diesem Falle ist es günstig, zur Ermöglichung der optischen Kontrolle der Dichtigkeit und Festigkeit der Verklebung, die Innenfläche des äußeren Schlauchansatzes 43 und die Außenfläche des inneren Schlauchansatzes 44 z. B. durch ein entsprechend aufgerauhtes Spritzgießwerkzeug mattiert auszubilden, um durch Beobachtung von nach der Verklebung eventuell zurückbleibenden milchigen Stellen die Beschaffenheit der Verklebung ermitteln zu können. Die Verklebung ist dann homogen und dicht, wenn die Schlauchansätze 43,44 makellos glasklar sind.

## Patentansprüche

1. Verbindungsstück aus glasklarem Kunststoff mit einem hülsenförmigen Schlauchansatz zum Ein- oder Aufstecken eines mit dem Schlauchansatz mittels eines Klebe-/Lösungsmittels zu verbindenden Schlauches aus glasklarem Kunststoff, insbesondere für medizinische Überleitungsgeräte, dadurch gekennzeichnet, daß der Schlauchansatz (15-18) auf der dem Schlauch (12,13) zugewandten Fläche (15a-18a) mattiert ist.

2. Verbindungsstück nach Anspruch 1, dadurch gekennzeichnet, daß die Mattierung aus einer gleichmäßigen Oberflächenaufrauhung gebildet ist.

## Claims

1. A connecting piece of clearly transparent plastics material comprising a jacket-shaped tube hub for inserting or slipping on a tube of clearly transparent plastics to be connected with said tube hub by means of an adhesive agent or a solvent, respectively, in particular for medical transfer apparatus, characterised in that said tube hub (15–18) is frosted on the surface (15a–18a) facing said tube (12, 13).

2. The connecting piece according to claim 1, characterised in that said frosting is formed by a uniform roughening of the surface.

## Revendications

1. Elément de connexion en matière plastique transparente comportant un embout de raccordement de tuyau en forme de manchon, destiné à recevoir ou à s'emmancher sur un tuyau en matière plastique transparente devant être relié, au moyen d'un colle ou d'un solvant, à l'embout de raccordement, notamment destiné à des systèmes de tubulures de transfert d'usage médical, caractérisé en ce que l'embout de raccordement de tuyau (15–18) est dépoli sur sa surface (15a–18a) tournée vers le tuyau (12, 13).

2. Elément de connexion selon la revendication 1, caractérisé en ce que le dépoli est formé par une rugosité uniforme de l'état de surface.

EP 0 279 925 B1

FIG.1

FIG.2

FIG.3

FIG.4

FIG.5

30

37          35       36       32

FIG.6

31

38       39

42

40

FIG.7

43

44

41